Europäisches Patentamt

European Patent Office

Office européen des brevets

(11) Veröffentlichungsnummer: **0 248 246**
**B1**

(12) **EUROPÄISCHE PATENTSCHRIFT**

(45) Veröffentlichungstag der Patentschrift: **19.09.90**

(21) Anmeldenummer: **87107044.7**

(22) Anmeldetag: **15.05.87**

(51) Int. Cl.⁵: **A 61 F 2/06, A 61 L 27/00**

(54) Künstliche Gefässwand.

(30) Priorität: **02.06.86 CH 2220/86**

(43) Veröffentlichungstag der Anmeldung:
**09.12.87 Patentblatt 87/50**

(45) Bekanntmachung des Hinweises auf die
Patenterteilung:
**19.09.90 Patentblatt 90/38**

(84) Benannte Vertragsstaaten:
**AT BE DE ES FR GB IT NL SE**

(56) Entgegenhaltungen:
**EP-A-0 130 401**
**EP-A-0 183 365**
**WO-A-82/03764**
**AU-A- 31 212**
**FR-A-2 391 709**

(73) Patentinhaber: **GEBRÜDER SULZER**
**AKTIENGESELLSCHAFT**
**Zürcherstrasse 9**
**CH-8401 Winterthur (CH)**

(72) Erfinder: **Turina, Marko, Prof. Dr.-med.**
**In den Looren 40**
**CH-8053 Zürich (CH)**
Erfinder: **Bittmann, Peter, Dr.**
**Langackerstrasse 126**
**CH-8704 Herrliberg (CH)**

(74) Vertreter: **Sparing Röhl Henseler Patentanwälte**
**European Patent Attorneys**
**Rethelstrasse 123**
**D-4000 Düsseldorf 1 (DE)**

Courier Press, Leamington Spa, England.

**Beschreibung**

Die Erfindung betrifft eine künstliche Gefässwand, insbesondere für arterielle Gefässe mit Innendurchmessern kleiner 7 mm, bestehend aus einer — mindestens für Wasser, Nährstoffe und Stoffwechselprodukte des Gewebes — semipermeablen Membran aus bioinerten Kunststoff. Aus der EP—A—0 130 401 ist eine Künstliche Gefäßwand, insbesondere für arterielle Gefäße mit kleinem Durchmesser, bekannt, die eine durchlässige Membran aus einem bioinerten Kunststoff aufweist.

Aus der Erfahrung ist bekannt, dass für Flüssigkeiten — vor allem für Wasser, Nährstoffe, Gewebeflüssigkeit und Stoffwechselprodukte — undurchlässige Substanzen als künstliche Gefässwände ungeeignet sind, da die Bewegung der Gewebeflüssigkeit dadurch weitgehend behindert wird, was zu Störungen des physiologischen Gleichgewichts und zu heftigen Reaktionen des Körpers führt. Es ist daher zu fordern, das künstliche Gefässwände für Flüssigkeiten mindestens teildurchlässig sind.

Ein Arterienersatz im Bereich grosser Gefässe — beispielsweise der Aorta bis zur Leistengegend — gilt als weitgehend gelöst, beispielsweise durch textile Prothesen oder durch Prothesen aus expandiertem Polytetrafluoräthylen. Bei diesen "grossen" Gefässen wird die erwähnte Durchlässigkeit der Gefässwand durch den textilen Aufbau oder durch den Reckprozess erreicht, wobei gleichzeitig durch von aussen einwachsendes Gewebe eine Fixierung erzielt wird.

Kleinlumige Gefässprothesen der genannten Art mit einem Innendurchmesser unterhalb 7 mm neigen hingegen immer noch zum Verschluss. Die Wechselwirkung zwischen Blut und Kunststoff führt zur Bildung einer Fibrinschicht, die eine Lumen-Verengung zur Folge hat. Durch das dadurch verlangsamt fliessende Blut wird eine weitere Ablagerung begünstigt und es kommt zur Thrombosierung der Prothese. Zur Ueberwindung dieser Schwierigkeit sind Glandula-Prothesen bekannt (DE—PS 34 22 639), bei denen vor der Implantation die Innenseite mit einer Monoschicht patienteneigener Endothel-Zellen lückenlos ausgekleidet ist.

Eine ausreichende Durchlässigkeit haben an sich flüssigkeitsdurchlässige Gele — beispielsweise wässrige Gele auf Agar-Agar- oder Polyacrylamid-Basis —. Diese Gele sind als Gefässwände jedoch ungeeignet, da sie keine ausreichende mechanische Festigkeit haben, die beispielsweise den Druckpulsationen arterieller Blutströme zu widerstehen vermag.

Als durchlässige künstliche Wände, die die geschilderten Forderungen erfüllen, bieten sich elastische Membranen aus bioinertem Kunststoff an; derartige Membranen bestehen beispielsweise aus einem porenlosen Polymer von Polyaminosäuren und Urethan. Als geeignet haben sich auch Membranen von 0,2 bis 1 mm Dicke erwiesen, die aus Kunststoff, beispielsweise aus einem Polyurethan bestehen, bei dem die Durchlässigkeit durch durchgehend offene Poren gewährleistet wird. Da gleichzeitig eine Fixierung durch aussen ein- oder anwachsendes Gewebe angestrebt wird, sind Porengrössen von mindestens 10 bis 50 µm, vorzugsweise von 30 µm, zu fordern. Falls notwendig, ist es auch denkbar, die mechanische Festigkeit der genannten Membran durch eine "Armierung", beispielsweise aus bioinertem Fasermaterial, zu vergrössern.

Für die Haftung und Lebensfähigkeit einer Monoschicht aus Endothel-Zellen ist es notwendig, dass die Endothel-Zellen einen geschlossenen zusammenhängenden Belag bilden. Ihre Haftung auf dem Kunststoff, auf dem sie unter Bildung einer Basalmembran aus Kollagen, Typ IV, wachsen, muss dabei so gross sein, dass sie vom Blutstrom nicht mitgerissen werden.

Aufgabe der Erfindung ist es, die Regeneration, Haftung und Lebensfähigkeit einer einlagigen Schicht aus Endothel-Zellen auf einer mechanisch stabilen, elastischen Gefässwand kleinlumiger Gefässe zu verbessern. Die Lösung dieser Aufgabe erfolgt erfindungsgemäss dadurch, dass die Membran blutstromseitig einlagig mit einer Schicht lebender Endothel-Zellen und auf der Aussenseite mit lebenden Zellen einer glatten, elastische Fasern erzeugenden Muskulatur (SMC) belegt ist.

Zwischen den Endothel-Zellen und den Muskelzellen, die ein- oder mehrschichtig — gleichzeitig mit den Endothel-Zellen oder in getrennten Arbeitsgängen — auf die äussere Oberfläche der Membran aufgebracht sein können, ergeben sich so Wechselwirkungen biochemischer und physiologischer Art, die zu einer Verbesserung der Lebensfähigkeit der lebenden Zellen auf der Kunststoffmembran führen. Die von den Muskelzellen gebildeten elastischen Fasern verstärken darüberhinaus die Membran als Gefässwand und sichern die Elastizität auch wenn der Kunststoff der Membran langzeitig unter Umständen Veränderungen erleidet.

Werden die Gefässwände aus Kunststoff mit Mikroporen mit den erwähnten Durchmessern gefertigt, so ist es vorteilhaft, wenn die Membran mindestens auf ihrer blutstromseitigen Oberfläche derart geglättet ist, dass einerseits ihre Permeabilität mindestens teilweise erhalten bleibt, andererseits jedoch eine geschlossene Monoschicht von Endothel-Zellen wächst. Denn bei Verwendung von derartigen Membranen hat sich gezeigt, dass derart grosse "Löcher" in der Kunststoffoberfläche von den Endothel-Zellen nur schwer oder gar nicht überwachsen werden können, so dass eine geschlossene Schicht von Endothel-Zellen, wenn überhaupt, nur schwer gezüchtet werden kann. Durch das "Glätten" wird die Oberfläche der Membran mindestens auf ihrer Innenseite jedoch durch Füllen oder Abdecken der "grossen" Poren so vorbereitet, dass die Endothel-Zellen diese Störstellen der Oberfläche gut überwachsen können, so dass sie eine geschlossene, zusammenhängende und fest haftende Monoschicht bilden.

Mikroporöse Membranen können beispielsweise nach dem bekannten Verfahren der Phasentrennung bei niedrigen Temperaturen hergestellt werden.

Für die "Glättung" der Innenwand-Oberfläche können mit Vorteil zwei Wege beschritten werden. Zum einen ist es möglich, dass die Mikroporen mit einem wässrigen Gel gefüllt sind, das für Moleküle mit einem Molekulargewicht bis zu 100'000 Dalton permeabel ist; als wässrige Gele eignen sich dafür beispielsweise die bereits erwähnten Agar-Agar- oder Polyacrylamid-Gele.

Andererseits kann man mindestens die blutstromseitigen Oberflächen der Mikroporen auch mit einer porösen Schicht schliessen, bei der der Durchmesser der Poren mindestens annähernd mit den Abmessungen von Endothel-Zellen vergleichbar ist; hier lassen sich beispielsweise Membranen verwenden, wie sie aus dem Gebiet der Ultrafiltration bekannt sind.

Natürliche Gefässwände sind bekanntlich so ausgebildet, dass sie bei Erhöhungen des Innendruckes eine elastische Dehnung aufweisen, die mit steigender Druckerhöhung einem oberen Grenzwert zustrebt. Kunststoffmembranen besitzen diese Eigenschaften des natürlichen Gewebes nur unvollkommen. Es ist daher zweckmässig, die Dehnbarkeit der Membran mit Hilfe einer stützenden Struktur zu begrenzen, die vorteilhafterweise aus textiler Maschenware besteht, deren Maschenweite zwischen 0,2 und 2 mm beträgt. Dabei hat sich besonders eine stützende Struktur aus einem Geflecht einzelner spiralförmig angeordneter Monofilament aus Polyester-Fäden von 10 bis 40 µm Durchmesser bewährt. Das Geflecht, dessen einzelne Filamente praktisch unelastisch sind, erhält eine gewisse Elastizität durch den textilen Aufbau, bei dem sich die einzelnen Filamente bei Druckänderungen relativ zueinander umlagern. In diesem Zusammenhang ist durch experimentelle Versuche ermittelt worden, dass die lineare Dehnbarkeit der künstlichen Gefässwand in einem Druckbereich des Innendruckes von 80 bis 150 mm Hg mit Vorteil 0,03 bis 0,1% pro mm Hg Druckerhöhung betragen kann. Der Spiralförmige Aufbau des Geflechtes ist knicksicher und verleiht so der Membran eine erhöhte Knickstabilität.

Im folgenden wird die Erfindung anhand eines Ausführungsbeispiels im Zusammenhang mit der Zeichnung näher erläutert.

Fig. 1 zeigt schematisch und vergrössert — ohne dass die einzelnen Elemente massstabgerecht zueinander dargestellt sind — einen Schnitt durch eine erste Ausführungsform der neuen Gefässwand;

Fig. 2 ist ein zweites Ausführungsbeispiel.

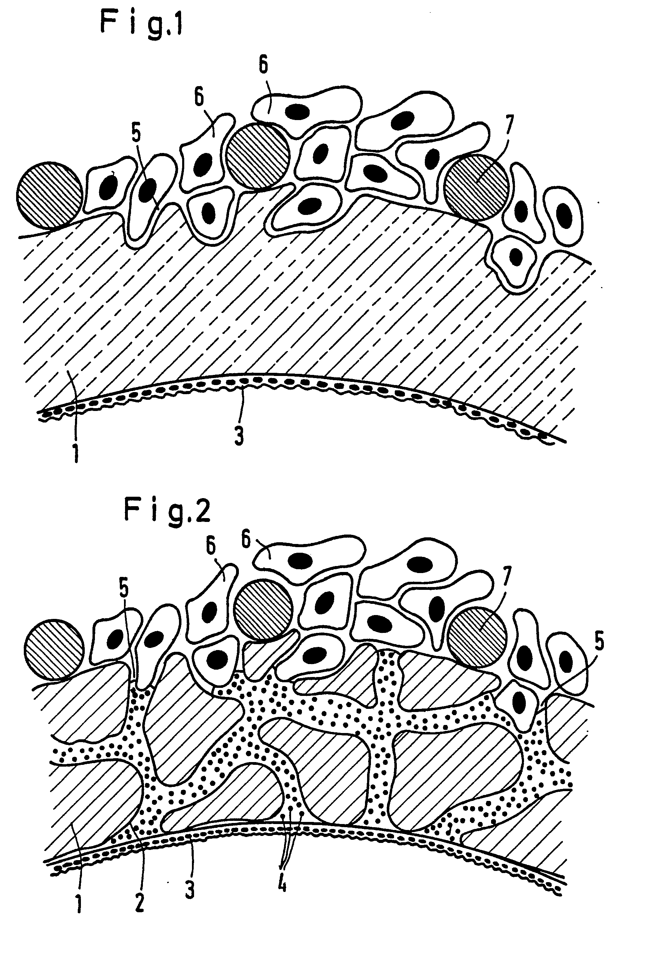

Das "Wandelement" der neuen Gefässwand bildet eine semipermeable Membran 1 (Fig. 1), vorzugsweise aus einem der genannten Kunststoffe. Sie hat beispielsweise eine Dicke zwischen 0,2 und 1 mm. Die innere Oberfläche der Membran 1 ist mit einer Monoschicht 3 von lebenden Endothel-Zellen belegt. An der äusseren Oberfläche der Membran 1 sind Hohlräume 5 vorhanden, in die — zur Fixierung des künstlichen Gefässes im Körper — Zellen 6 einwachsen können. Die Zellen 6 bestehen erfindungsgemäss aus glatten Muskelzellen (SMC: smooth muscle cells), die ebenfalls auf bekannte Weise gezüchtet und auf die Membran oder Gefässwand 1 ein- oder mehrschichtig aufgebracht werden. Diese glatten Muskelzellen 6 haben einerseits die Aufgabe, durch physiologische und biochemische Wechselwirkungen durch die Membran 1 hindurch die "Verträglichkeit" des künstliches Gefässes im Körper und damit die Lebensfähigkeit und die Vitalität der Endothel-Zellen 3 zu fördern; zum anderen bilden sie elastische Muskelfasern, die die mechanische Stabilität des künstlichen Gefässes erhöhen. Diese Wechselwirkungen werden ausser durch die Durchlässigkeit auch durch die Elastizität der Kunststoff-Membran 1 ermöglicht, die die Druckimpulskurven des Blutstromes an die Muskelzellen 6 weitergibt und ihre Produktion elastischer Fasern (Elastin) anregt.

Wie bereits erwähnt, ist die Dehnbarkeit natürlicher Gefässe und diejenige der Endothel-Zellen begrenzt; um eine ähnliche Begrenzung der Dehnung für eine mit festhaftenden Endothel-Zellen belegte künstliche Gefässwand aus einer Kunststoffmembran 1 zu erreichen, sind über die äussere Oberfläche der Membran 1 als stützende, nur wenig elastische Struktur einzelne Fäden 7 verteilt. Diese Fäden 7 sind beispielsweise unelastische Monofilamente aus Polyester mit Durchmesser von 10 bis 40 µm. Für ein in sich peripher geschlossenes, aderartiges, künstliches Gefäss besteht die Stützstruktur der Fäden 7 beispielsweise aus einem Geflecht, dessen Monofilamente spiralförmig verlaufen, und das seine begrenzte Elastizität aufgrund der mechanischen Verschiebbarkeit der einzelnen Fäden 7 erhält. Die erwähnten Dicken der Fäden 7 und Maschenweiten des Geflechts von beispielsweise 0,2 bis 2 mm ermöglichen es, die lineare Dehnung bei einem Innendruck im physiologischen Bereich zwischen 80 bis 150 mm Hg auf 0,03 bis 0,1% pro mm Hg Druckerhöhung zu begrenzen.

Bei dem Ausführungsbeispiel nach Fig. 2 ist die Permeabilität der Membran 1, die in diesem Fall aus einem Polyurethan besteht und beispielsweise nach dem Verfahren der Phasentrennung bei niedrigen Temperaturen gefertigt ist, durch ein Netz von Mikroporen 2 erzeugt, deren Durchmesser zumindest vorwiegend beispielsweise 30 µm betragen. Wie Fig. 2 zeigt, sind die Mikroporen 2 sowohl an der äusseren als auch an der inneren Oberfläche der Membran 1 offen.

Da die Endothel-Zellen — wie geschildert — Hohlräume, deren Durchmesser ein Mehrfaches ihrer eigenen Abmessungen betragen, im allgemeinen nicht oder nur schwer überwachsen können, ist die innere Oberfläche der Membran 1 "geglättet". Diese Glättung erfolgt in dem gezeigten Beispiel durch eine Füllung der Mikroporen 2 mit einem, die Permeabilität der Membran 1 nicht unzulässig beeinträchtigenden wässrigen Gel 4. Dieses Gel 4, das beispielsweise ein 6 %-iges Agarose- oder ein 12 bis 18 %-iges Polyacrylamid-Gel sein kann, wird beispielsweise nach einem bekannten Tauchverfahren in die Mikroporen 2 eingefüllt. Nach dem Tauchen wird das Gel an der äusseren Oberfläche der Membran 1 teilweise wieder herausgelöst, um die Hohlräume 5 für das Einwachsen der Zellen 6 zu schaffen. Die Herstellung der "Füll"-Gele kann durch verschiedene miteinander kombinierte bekannte Verfahrensschritte erfolgen. Im Rahmen dieser bekannten Verfahren wird das Agarose-Gel darüberhinaus in ebenfalls bekannter Weise im Hinblick auf ein verbessertes Anwachsen von lebenden Zellen durch eine elektrische Oberflächenbeladung konditioniert.

Eine Sterilisation der zu implantierenden Gefässwand bzw. ihrer Einzelteile erfolgt beispielsweise auf chemischem Weg durch Einlegen in eine Sterilisationslösung. Die Dicke der Kunststoffmembran soll für die angestrebten Wechselwirkungen möglichst gering und für eine leichte Handhabung durch den Chirurgen möglichst gross sein.

## Patentansprüche

1. Künstliche Gefässwand, insbesondere für arterielle Gefässe mit Innendurchmessern kleiner 7 mm, bestehend aus einer semipermeablen Membran aus bioinerten Kunststoff, welche Membran blutstromseitig einlagig mit einer Schicht lebender Endothel-Zellen und auf der Aussenseite mit lebenden Zellen einer glatten, elastische Fasern erzeugenden Muskulatur (SMC) belegt ist.

2. Gefässwand nach Anspruch 1, dadurch gekennzeichnet, dass die Membran aus einem porenlosen Polymer von Polyaminosäuren und Urethan besteht.

3. Gefässwand nach Anspruch 1, dadurch gekennzeichnet, dass die Membran aus einem Kunststoff mit durchgehend offenen Mikroporen besteht, deren Durchmesser mindestens teilweise zwischen 10 und 50 μm, vorzugsweise bei 30 μm, liegen.

4. Gefässwand nach Anspruch 3, dadurch gekennzeichnet, dass die Membran mindestens auf ihrer blutstromseitigen Oberfläche derart geglättet ist, dass einerseits ihre Permeabilität mindestens teilweise erhalten bleibt, andererseits jedoch eine geschlossene Monoschicht von Endothel-Zellen wächst.

5. Gefässwand nach Anspruch 1, dadurch gekennzeichnet, dass die Dehnbarkeit der Membran mit Hilfe einer stützenden Struktur begrenzt ist.

6. Gefässwand nach Anspruch 5, dadurch gekennzeichnet, dass bei einem Innendruck von 80 bis 150 mm Hg die lineare Dehnung der Membran 0,03 bis 0,1% pro mm Hg Druckerhöhung beträgt.

7. Gefässwand nach Anspruch 5, dadurch gekennzeichnet, dass die stützende Struktur aus textiler Maschenware besteht, deren Maschenweite zwischen 0,2 und 2 mm beträgt.

8. Gefässwand nach Anspruch 7, dadurch gekennzeichnet, dass die stützende Struktur aus einem Geflecht einzelner spiralförmig angeordneter Filamente aus Polyester-Fäden von 10 bis 40 μm Durchmesser besteht.

## Revendications

1. Paroi vasculaire artificielle, notamment pour vaisseaux artériels ayant des diamètres intérieurs de moins de 7 mm, constituée d'une membrane semi-perméable en matière plastique bio-inerte, cette membrane étant tapissée, du côté de l'écoulement sanguin, d'une monocouche de cellules endothéliales vivantes et étant revêtue, du côté extérieur, de cellules vivantes d'un tissu musculaire lisse (SMC) produisant des fibres élastiques.

2. Paroi vasculaire suivant la revendication 1, caractérisée en ce que la membrane est constituée d'un polymère non poreux de polyaminoacides et d'uréthanne.

3. Paroi vasculaire suivant la revendication 1, caractérisée en ce que la membrane est en une matière plastique à pores microscopiques ouverts aux deux extrémités, dont les diamètres se situent au moins partiellement entre 10 et 50 μm, de préférence 30 μm.

4. Paroi vasculaire suivant la revendication 3, caractérisée en ce que la membrane est rendue lisse au moins à sa surface tournée vers le courant sanguin de manière que, d'une part, sa perméabilité soit au moins partiellement maintenue mais que, d'autre part, une monocouche fermée de cellules endothéliales se développe.

5. Paroi vasculaire suivant la revendication 1, caractérisée en ce que l'extensibilité de la membrane est limitée à l'aide d'une structure de soutien.

6. Paroi vasculaire suivant la revendication 5, caractérisée en ce que l'allongement linéaire de la membrane atteint 0,03 à 0,1% par mm de Hg d'élévation de pression pour une pression interne de 80 à 150 mm de Hg.

7. Paroi vasculaire suivant la revendication 5, caractérisée en ce que la structure de soutien est constituée d'une matière textile à mailles dont l'ouverture de maille se situe entre 0,2 et 2 mm.

8. Paroi vasculaire suivant la revendication 7, caractérisée en ce que la structure de soutien est formée d'un réseau entrelacé de filaments individuels à disposition hélicoïdale, formés de fils en polyester de 10 à 40 μm de diamètre.

## Claims

1. An artificial vessel wall, more particularly for arterial vessels of less than 7 mm lumen, comprising a semi-permeable diaphragm of a bioinert plastics, which diaphragm has on the blood flow side a single layer of a coating of living endothelium cells and on the outside has a coating of living smooth muscle cells (SMC) producing resilient fibres.

2. A vessel wall according to claim 1, characterised in that the diaphragm is made of a pore-less polymer of polyaminoacids and urethane.

3. A vessel wall according to claim 1, characterised in that the diaphragm is made of a plastics with throughout open micropores whose diameters are at least to some extent between 10 and 50 µm, preferably 30 µm.

4. A vessel wall according to claim 3, characterised in that the diaphragm is so smoothed at least on its surface near the blood flow that its permeability is retained at least to some extent but a closed monolayer of endothelium cells grows.

5. A vessel wall according to claim 1, characterised in that the stretchability of the diaphragm is limited by means of a support structure.

6. A vessel wall according to claim 5, characterised in that the linear stretch of the diaphragm in response to an inner pressure of from 80 to 150 mm Hg is from 0.03 to 0.1% per mm Hg of pressure increase.

7. A vessel wall according to claim 5, characterised in that the support structure consists of textile mesh material whose mesh size is between 0.2 and 2 mm.

8. A vessel wall according to claim 7, characterised in that the support structure consists of a braiding of discrete spirally arranged filaments of polyester threads of from 10 to 40 µm in diameter.

# Fig.1

# Fig.2